Europäisches Patentamt

European Patent Office

Office européen des brevets

(11) Numéro de publication : **0 330 641 B1**

(12) **FASCICULE DE BREVET EUROPEEN**

(45) Date de publication du fascicule du brevet :
28.07.93 Bulletin 93/30

(51) Int. Cl.⁵ : **C12N 9/20,** C12N 9/52, C11D 3/386, // (C12N9/20, C12R1:38)

(21) Numéro de dépôt : 89870029.9

(22) Date de dépôt : 20.02.89

(54) Nouvelle lipase et compositions détergentes en contenant.

(30) Priorité : 22.02.88 BE 8800207

(43) Date de publication de la demande :
30.08.89 Bulletin 89/35

(45) Mention de la délivrance du brevet :
28.07.93 Bulletin 93/30

(84) Etats contractants désignés :
AT BE CH DE ES FR GB GR IT LI LU NL SE

(56) Documents cités :
EP-A- 0 214 761
EP-A- 0 218 272
CHEMICAL ABSTRACTS, vol. 109, no. 4, 25 juillet 1988, page 81, résumé no. 24544a, Columbus, Ohio, US

(56) Documents cités :
CHEMICAL ABSTRACTS, vol. 101, no. 25, 17 décembre 1984, page 320, résumé no. 225392h, Columbus, Ohio, US; G.L. CHRISTEN et al.: "Selected properties of lipase and protease of Pseudomonas fluorescens 27 produced in four media"

(73) Titulaire : FINA RESEARCH S.A.
Zone Industrielle C
B-7181 Seneffe (BE)

(72) Inventeur : Paridans, Line
Rue Camille Duray, 5
B-7190 Ecaussines (BE)
Inventeur : Tirtiaux-Nafpliotis, Léa
Rue des Combattants, 162
B-1301 Bierges (BE)

(74) Mandataire : Leyder, Francis et al
c/o Fina Research S.A. Dépt. Brevets Zone Industrielle C
B-6520 Feluy (BE)

## Description

La présente invention se rapporte à une nouvelle lipase ayant d'excellentes propriétés de résistance à la température et une grande stabilité dans une large gamme de pH ainsi qu'une stabilité chimique accrue envers divers agents de dénaturation.

Les lipases sont une classe d'enzymes fortement recherchées ces dernières années, en fonction de leur utilité dans de nombreuses applications, notamment en oléochimie ou dans la composition de savons et de détergents.

Un grand nombre d'enzymes qui sont connues pour appartenir à la classe des lipases ne sont pas suffisamment efficaces dans les détergents, à cause de la faible stabilité de ces enzymes dans ces milieux dénaturants. Ceci a conduit à la recherche et au développement d'enzymes plus stables en solution détergente.

La nouvelle lipase de la présente invention peut être isolée de microorganismes lipasiques, en particulier d'une souche Pseudomonas décrite ci-après, et présente des caractéristiques physiques et chimiques particulières.

La lipase de l'invention est caractérisée par ses propriétés physiques et chimiques comprenant à la fois :
- un poids moléculaire d'environ 55000 daltons,
- un point isoélectrique inférieur à 4,
- la capacité d'hydrolyser et de synthétiser des glycérides et des esters,
- une activité non spécifique quant à la nature de l'acide gras,
- une activité maximale entre pH 6 et pH 8, dont au moins 50% est exprimée entre pH 5,5 et pH 10,
- la stabilité à 30°C pendant 30 minutes au moins de pH 3 à pH 11,5 et pendant 150 minutes au moins de pH 4,5 à pH 10,5,
- une activité à 30°C supérieure à 30% de l'activité maximale observée à plus haute température,
- la stabilité à la température caractérisée par une conservation d'activité supérieure à 75% à 60°C pendant 30 minutes,
- une activité pratiquement égale en solution détergente à celle exprimée dans l'eau dans les mêmes conditions, et
- la capacité d'enlever des taches en solution détergente, même en présence d'additifs oxydants;

ces propriétés étant déterminées comme on l'explique ci-après.

Bien que l'on décrive ici la lipase produite par la souche Pseudomonas décrite ci-après, il est entendu que l'invention concerne également toute lipase ayant les caractéristiques mentionnées précédemment, y compris la lipase que l'on pourrait produire à l'aide d'une souche d'un micro-organisme qui aurait été manipulée génétiquement à cet effet.

Une souche Pseudomonas produisant la lipase de l'invention a été obtenue par sélection dirigée de micro-organismes sauvages prélevés dans des eaux résiduaires chaudes contenant principalement des matières grasses comme substrat. Cette souche n'appartient pas aux variétés pathogènes connues de Pseudomonas.

Cette souche a été déposée le 8 février 1988 sous le numéro CBS 134.88 à l'Institut voor Schimmelcultures à Baarn aux Pays-Bas.

Les conditions de culture de ce micro-organisme qui produit la lipase de l'invention ne sont pas critiques parce que la production de lipase est constitutive, c'est-à-dire que le micro-organisme en produit même en l'absence de substrat gras. Ces conditions de culture peuvent donc être adaptées pour produire un maximum d'enzyme.

On utilisé également des conditions de culture qui comprennent un milieu minimum (tampon phosphate 50 mM, sulfate magnésique 0,1 g/l, citrate trisodique 0,5 g/l), et un substrat non gras d'une des deux catégories suivantes :
- soit un saccharide (glucose, lactose, fructose, saccharose; 10 g/l) auquel on ajoute $NH_4^+$ (sulfate ammonique, 1 g/l) pour produire la lipase
- soit un substrat complexe (peptone 15 g/l, extrait de levure 15 g/l ou mélasse 30 g/l) pour produire la lipase et une protéase qui est entièrement compatible avec cette lipase.

Les conditions de culture comprennent généralement un pH de 5 à 10, de préférence de 6 à 7, une température comprise entre 15 et 40°C,
de préférence environ 37°C, et une aération suffisante.

Le lipase est produite pendant la croissance du micro-organisme et directement excrétée dans le milieu. On récupère la lipase selon les techniques habituelles, comprenant généralement une centrifugation pour éliminer le micro-organisme, puis une concentration par ultrafiltration suivie d'un désalage. On peut ensuite sécher la lipase brute ainsi obtenue; on peut également procéder à une étape supplémentaire de purification pour séparer la lipase de protéines contaminantes, dont la protéase lorsqu'elle a été produite. En effet, si le milieu de culture contenait des peptides, la souche excrète également une protéase que l'on retrouve dans la lipase brute.

Bien qu'en règle générale on puisse indifféremment produire la lipase en cultivant la souche <u>Pseudomonas</u> en batch ou en continu, on observe parfois que l'activité lipasique, mesurée dans le milieu de culture, passe par un maximum (par exemple en utilisant le glucose ou la mélasse comme substrat). Dans ce cas, on sait qu'il est préférable de travailler en fermenteur continu.

Enfin, il est également possible de cultiver la souche <u>Pseudomonas</u> en l'absence d'oxygène, à condition d'utiliser un nitrate comme accepteur terminal d'électrons.

Le lipase de l'invention présente une activité hydrolytique et synthétique non seulement vis-à-vis des glycérides, mais également vis-à-vis d'autres esters. Cette activité n'est pas spécifique quant à la nature de l'acide gras.

L'activité de la lipase de l'invention a été déterminée par les méthodes suivantes :

### a) dosage colorimétrique

Dans un tube de micro-centrifugeuse, on introduit 0,5 ml de milieu réactionnel (tampon phosphate sodique 100 mM à pH 8.0 contenant 2,3 mg/ml de déoxycholate sodique et 1,1 mg/ml de gomme arabique) et 0,4 ml de l'échantillon à doser, que l'on incube à la température désirée (45°C sauf mention contraire). On initie la réaction enzymatique par addition de 0,1 ml d'une solution fraîchement préparée de substrat (3 mg/ml de p-nitrophénylpalmitate dans l'isopropanol) et on homogénéise immédiatement. Après incubation (20 minutes en principe, ou moins si la coloration jaune se développe rapidement), on stoppe la réaction enzymatique par addition de 0,5 ml HCl 3N. Après centrifugation (en générale 15 minutes à 13000 rpm), on prélève 1 ml du surnageant que l'on mélange avec 3 ml NaOH 2N. On lit extemporanément la densité optique (qui doit être comprise entre 0,1 et 0,8) à 400 nm, en utilisant de l'eau comme témoin. La valeur de l'activité lipasique est obtenue à partir d'une droite d'étalonnage. Elle est exprimée en unités enzymatiques (U.E.) correspondant à 1 micromole de p-nitrophénol (et par conséquent 1 micromole d'acide gras) par heure dans les conditions de l'essai.

Le dosage colorimétrique est sensible (0,1 U.E. au moins) fiable et rapide. Il a l'avantage d'être réalisé en milieu aqueux, plus proche des conditions d'utilisation des compositions détergentes.

### b) Dosage titrimétrique

On mélange 6 ml d'huile de soja et 4 ml de l'échantillon à doser en présence de chlorure de magnésium 10 mM, et on incube le mélange à la température désirée, sous forte agitation, pendant 60 minutes. Après centrifugation (en général 5 minutes à 7000 rpm), on prélève une partie aliquote de la phase supérieure, on y ajoute 20 ml d'alcool dénaturé neutralisé et on titre sous agitation par une solution de NaOH 0,03 N en présence de phénolphtaléine. L'activité est également exprimée en UE.

Le dosage titrimétrique a l'avantage de refléter l'activité de la lipase sur ses substrats naturels que sont les glycérides. Cependant, il ne fournit qu'une valeur relative (de surcroît non linéaire) dépendant de la nature de l'émulsion.

L'activité de la lipase de l'invention présente un maximum en forme de plateau entre pH 6 et pH 8 dans les conditions du dosage titrimétrique (tampons citrate + phosphate à pH 3-6, phosphate à pH 6-8, HCl-tris(hydroxyméthyl)aminométhane à pH 8-10); la lipase exprime au moins 50% de son activité maximale entre environ pH 5,5 et 10.

La stabilité au pH de la lipase de l'invention a été déterminée en maintenant la lipase à 30°C pendant 30 ou 150 minutes au pH désiré, puis en dosant l'activité lipasique résiduelle par colorimétrie. L'activité maximale est conservée pendant 30 minutes de pH 3 à pH 11,5, et pendant 150 minutes de pH 4,5 à pH 10,5. On observe encore 80% de l'activité maximale après 150 minutes à pH 12. Même en présence de 0,1% de Triton-X 100 (octylphénol polyéthoxylé; Rohm et Haas), on observe encore 75% de l'activité maximale après 45 minutes à pH 11.

L'activité de la lipase de l'invention dépend de la température. En utilisant la méthode titrimétrique (avec un substrat gras), on observe que l'activité est maximale à 60°C et que la lipase en exprime plus de 80% à 40°C. En utilisant la méthode colorimétrique (en milieu aqueux), le maximum d'activité s'exprime à 47°C, et la lipase en conserve plus de 60% à 40°C. Dans les deux cas, la lipase exprime encore à 30°C plus de 30% de l'activité maximale.

La lipase de l'invention est remarquablement stable en solution aqueuse aux températures habituelles de lavage. Après 30 minutes à 30, 40 ou 60°C, elle exprime respectivement 100, 95 et 75% de l'activité de départ, les mesures étant réalisées par la méthode colorimétrique.

En solution détergente, l'activité de la lipase de l'invention est maximale pour une concentration d'environ 5 g de base détergente par litre. Après 30 minutes à 30°C dans des solutions détergentes à 5 g/l, la lipase exprime encore pratiquement toute son activité :

91% dans une solution à pH 9,4 de base détergente sans oxydant

84% dans une solution à pH 10,3 d'un détergent commercial (lessive domestique)

81% dans une solution à pH 11,1 d'un second détergent commercial

69% dans une solution à pH 8,3 d'un troisième détergent comercial.

La lipase de l'invention est également caractérisée par son activité pour l'enlèvement des taches en solution détergente, même en présence d'additifs oxydants.

Le poids moléculaire de la lipase de l'invention est d'environ 55 000, comme déterminé par tamisage moléculaire en présence de Triton X-100 (octylphénol polyéthoxylé; Rohm et Haas) pour désagréger les aggrégats sans dénaturer l'enzyme. Ce poids moléculaire est notablement plus élevé que celui de la majorité des autres lipases excrétées par des micro-organismes.

On a établi que le point isoélectrique de la lipase de l'invention est inférieur à 4.

Ainsi qu'on l'a expliqué ci-dessus, la souche <u>Pseudomonas</u> capable de produire la lipase de l'invention est susceptible de produire également une protéase. Cette protéase est entièrement compatible avec la lipase de l'invention; elle est également active en détergence. La protéase de l'invention n'est pas toujours produite. Lorsqu'elle l'est, elle est produite en même temps que la lipase de l'invention par la souche <u>Pseudomonas</u> décrite ci-dessus, et il est possible de les séparer par les techniques connues, par exemple par chromatographie d'exclusion.

Pour doser l'activité protéolytique, on a utilisé la méthode suivante :

Dans un tube à centrifugation, on introduit 1 ml de solution de substrat (0,5 g azocaséine dans 100 ml de tampon HCl-tris(hydroxyméthyl)aminométhane à 200 mM à pH 7) et 0,5 ml de la solution enzymatique à doser, adéquatement diluée. Après 30 minutes d'incubation à 37°C, on ajoute 2 ml d'une solution à 0,1 g/ml d'acide trichloroacétique, on homogénise soigneusement et on centrifuge 5 minutes à 5000 rpm. On prélève alors 1 ml du surnageant, que l'on ajoute à 2 ml de NaOH 250 mM. On lit la densité optique à 400 nM contre un témoin obtenue en remplaçant dans le test, l'échantillon à doser par de l'eau distillée. Pour assurer la linéarité de la méthode, les densités optiques lues doivent être comprises entre 0,05 et 0,2. Une unité protéolytique est la quantité d'enzyme qui provoque une augmentation de densité optique de 1.0 par rapport au témoin, dans les conditions du dosage. Cette unité correspond à environ 1/4000 d'unité protéolytique déterminée par la méthode d'ANSON.

Un autre aspect de l'invention concerne les compositions détergentes contenant la lipase et/ou la protéase de l'invention. Les compositions détergentes de l'invention peuvent être formulées sous forme de poudre ou de liquide, ou sous toute autre forme.

Ces compositions détergentes contiennent comme composant détergent actif un mélange d'au moins un détergent synthétique anionique et au moins un détergent synthétique non-ionique. Ces types de détergent sont bien connus de l'homme de l'art. En général, le rapport en poids détergent anionique/détergent non-ionique varie de 12:1 à 1:12, de préférence de 6:1 à 1:6. Le total de ces deux détergents dans la composition est habituellement de 1 à 30% en poids, de préférence de 6 à 25%. Ces compositions détergentes comprennent le plus souvent au moins un savon, représentant en général de 1 à 12% en poids. On peut également ajouter des détergents d'autres types, tels des détergents cationiques ou amphotères.

Les compositions détergentes comprennent généralement d'autres ingrédients usuels en teneurs usuelles, qui peuvent être exempts de phosphates.

Ainsi, les compositions en poudre peuvent contenir de 1 à 45% en poids, de préférence de 1 à 30%, d'un ou plusieurs agents structurants organiques et/ou inorganiques. Comme exemples typiques de tels agents structurants, on peut citer les ortho-, pyro- et tripolyphosphates alcalins, les carbonates alcalins, seuls ou en mélange avec de la calcite, les sulfates alcalins, les silicates alcalins, les citrates alcalins, les nitrilotriacétates alcalins, le carboxyméthylcellulose, les carboxyméthyloxysuccinates, les zéolites, les polyacétalcarboxylates. De plus, ces compositions peuvent contenir de 1 à 35 % en poids, de préférence de 20 à 30% d'un agent oxydant ou d'un système oxydant comprenant un agent oxydant et un activateur de celui-ci. Comme agent oxydant on utilise généralement le perborate de sodium.

Les compositions liquides sont généralement à base d'eau (environ 40% en poids); elles peuvent contenir jusqu'à 30% de solvants organiques, tels l'éthanol ou le propylène glycol, et contiennent généralement de 1 à 20% en poids, de préférence environ 10% d'au moins un composé basique tel que la triéthanolamine.

Ces compositions peuvent en sus contenir des agents de blanchiment optique, des renforcateurs de mousse, des limiteurs de mousse, des agents anti-corrosion, des agents complexants ou de mise en suspension, des parfums, des colorants, des agents stabilisants pour les enzymes et/ou pour les agents oxydants, ou tous autres additifs usuels.

La lipase de l'invention est ajoutée dans le composition détergente, à raison de 0,1 à 50% en poids de cette composition, de préférence de 0,5 à 10% éventuellement sous forme d'additif enzymatique dont le principal composant actif est la lipase de l'invention, et qui peut également contenir une protéase, celle-ci n'étant pas nécessairement celle de l'invention.

Un troisième aspect de l'invention concerne un procédé de lavage utilisant les détergents de l'invention. On utilise généralement une solution contenant de 1 à 20 g/l de composition détergente, de préférence de 3 à 7 g/l. Selon un mode d'exécution préféré de l'invention, le pH de la solution de lavage est compris entre 7 et 12, de préférence entre 8 et 11, et la température de lavage est inférieure ou égale à 60°C, de préférence comprise entre 30 et 40°C, et la durée du lavage est comprise entre 10 et 90 minutes, de préférence entre 25 et 45 minutes.

Selon un mode d'exécution préféré de l'invention, on introduit dans la composition détergente une quantité de lipase (ou d'additif enzymatique) de l'invention telle que l'activité lipasique dans la solution de lavage soit supérieure à 350 UE colorimétriques par ml (> 0,5 UE titrimétriques par ml); de préférence supérieure à 700 UE colorimétriques par ml (> 1 UE titrimétrique par ml). Des quantités plus faibles de lipase auront une influence négligeable sur le résultat du lavage. De préférence, on introduira la lipase en quantité telle que l'activité lipasique soit supérieure à 700 UE colorimétriques par ml (> 1 UE titrimétrique par ml); des quantités jusqu'à cinq fois plus importantes de lipase ne semblent avoir aucun effet favorable ou défavorable sur le résultat des lavages. Lorsque l'on introduit également une protéase dans la composition détergente, on préfère que l'activité protéolytique soit supérieure à 0,6 unités Anson/l, de préférence supérieure à 1 unité Anson par l.

La présente invention sera à présent décrite à l'aide des exemples suivants, qui sont donnés afin de mieux illustrer sans pour autant en limiter la portée.

### Exemple 1 (LP)

On a préparé un milieu de croissance et de production composé d'un tampon phosphate potassique 50 mM à pH 6, contenant 0,5 g/l de citrate trisodique, 0,1 g/l de sulfate magnésique et 15 g/l de peptone de caséine, que l'on a stérilisé à 121°C pendant 20 minutes.

Dans une fiole de 1000 ml, on a introduit 100 ml de milieu de production que l'on a ensemencé par une goutte de suspension contenant la souche de Pseudomonas (solution de glycérol à 40% contenant la souche et conservée à -80°C) et mise à incuber à 37°C pendant 24 heures sous agitation orbitale à 200 tours par minute (rpm).

Cette culture a servi à ensemencer un fermenteur de 6 litres, stérilisé in situ, à 121°C pendant 1 heure, contenant 4 litres de milieu de production. On a réglé l'agitation (valeur nominale : de 400 à 1000 rpm) et l'aération (valeur nominale : 6 l air par minute) de telle sorte que la pression en oxygène dissous dans le fermenteur soit égale au moins à 5% de la saturation, en ajoutant de faibles quantités d'agent anti-moussant de type silicone pour éviter tout débordement par moussage. On a incubé à 37°C pendant 10 heures.

Pendant les 4 premières heures, on a observée une phase exponentielle rapide de croissance de la souche, au terme de laquelle l'activité lipasique atteignait 122 UE colorimétriques par ml. Ensuite, on a observé une phase exponentielle lente de croissance de la souche, s'achevant après environ 9 heures, au cours de laquelle on a observé une importante excrétion de la lipase :
- après 6,5 heures, on a mesuré 375 UE colorimétriques par ml;
- après 9 heures, on a mesuré 740 UE colorimétriques par ml;
- après 10 heures, on a atteint un plateau à 800 UE colorimétriques.

Après incubation, la culture étant arrivée en phase stationnaire, les micro organismes ont été séparés par centrifugation. Le surnageant a été concentré et dialysé contre de l'eau par ultrafiltration (seuil de coupure 10.000 daltons) sur un appareil "hollow fibre" de marque AMICON jusqu'à un volume final de 0,1 litre.

L'activité lipasique de cette solution correspondait à 30 000 UE colorimétriques ou 109,5 UE titrimétriques par ml. Son activité protéolytique correspondait à 0,077 unités Anson par ml.

Par lyophilisation, on a obtenu 3,2 g d'extrait enzymatique brut, se présentant sous forme d'une poudre brune.

### Exemple 2 (LPC)

On a répété la procédure de l'exemple 1 jusqu'à obtenir une solution de 3,2 g d'extrait enzymatique brut dans 100 ml d'eau. A cette solution agitée à 0°C, on a ajouté lentement 200 ml d'acétone refroidie à la même température, puis on a continué à agiter pendant 15 minutes. Après centrifugation, on a recueilli le culot protéique, contenant la lipase, que l'on a remis en suspension dans un minimum d'eau distillée. Après une microfiltration pour éliminer particules et/ou débris cellulaires, on a lyophilisé la solution et recueilli 800 mg d'une poudre beige.

On a déterminé que cet échantillon de poudre contenait 0,5 mg de protéines par mg de poudre (par rapport à l'albumine bovine, en utilisant la méthode Folin). Cette poudre exprimait une activité lipasique de 3200 UE colorimétriques par mg, et une activité protéolytique de 0,082 unités Anson par mg.

Exemple 3 (LC)

On a dissous 100 mg de la poudre beige obtenue à l'exemple 2 dans 1 ml de tampon phosphate 50 mM à pH 7. On a fait passer cette solution sur une colonne de chromatographie d'exclusion (Sephadex G-150, diamètre 26 mm, longueur 40 cm) et on a élué avec un tampon phosphate 50 mM à pH 7.

Le lipase étant exclue, ainsi que la majorité des autres constituants qui forment des aggrégats avec la lipase, elle est éluée immédiatement après le volume mort de la colonne, exempte de protéase qui est éluée plus tard et recueillie séparément.

Après lyophilisation des deux solutions on a recueilli 92 mg de poudre beige contenant la lipase et 2 mg de poudre blanche contenant la protéase.

La poudre contenant la lipase exprimait 3420 UE colorimétriques par mg, tandis que celle contenant la protéase exprimait 4.0 unités Anson par mg.

Exemple 4 (L)

On a prépare un milieu de croissance (stérilisé à 121°C pendant 20 minutes) composé d'un tampon phosphate potassique 50 mM à pH 7, contenant 0,5 g/l de citrate trisodique, 0,1 g/l de sulfate magnésique, 1 g/l de sulfate ammonique et 15 g/l de glucose (stérilisé séparément).

Dans une fiole de 1000 ml, on a introduit 100 ml du milieu de croissance que l'on a ensemencé par une goutte de suspension contenant la souche de Pseudomonas (solution de glycérol à 40% contenant la souche et conservée à -80°C) et mise à incuber à 37°C pendant 24 heures sous agitation orbitale à 220 rpm.

Cette culture a servi à ensemencer un fermenteur de 2 litres, stérilisé in situ à 121°C pendant 1 heure, contenant 1,5 litres de milieu stérile de production (identique au milieu stérile de croissance sauf qu'il ne contenait que 0,35 g/l de sulfate ammonique et 5 g/l de glucose).

On a démarré l'incubation à 37°C, avec une aération de 5 l d'air par minute en surface et une agitation d'environ 500 rpm régulée de telle sorte que la pression en oxygène dissous dans le fermenteur soit égale à au moins 1% de la saturation.

Après 7 heures, on a atteint une population de $3,4.10^{10}$ cellules par ml de milieu, et une activité lipasique dans ce milieu correspondant à 60 UE colorimétriques par ml.

A ce stade, on a amorcé le procédé en continu : on a introduit du milieu stérile de production avec un débit de 500 ml/h, tout en prélevant une quantité équivalente de culture, pendant 72 heures.

On a recueilli ainsi 36 litres de culture. Les micro organismes ont été séparés par centrifugation. On a recueilli le surnageant, que l'on a concentré et dialysé contre de l'eau par ultrafiltration sur un appareil constitué de deux modules en boucle, à seuil de coupure de 10000 Daltons, jusqu'à un volume de 5 litres. On a poursuivi la concentration sur un appareil "hollow fibre" AMICON jusqu'à un volume final de 1 litre.

L'activité lipasique de cette solution correspondait à 2000 UE colorimétriques par ml; elle était dépourvue de toute activité protéolytique.

Par lyophilisation, on a obtenu 40 g d'extrait enzymatique brut.

Exemple 5

L'activité enzymatique de la lipase de l'invention en solution détergente a été évaluée sur des tissus standards EMPA (Eidgenössische Materialprüfungs - und Versuchsanstalt, St Gallen, CH) avec salissures normalisées :
- n° 104 : tissu polyester/coton 65/35 sali d'huile d'olive et noir de carbone
- n° 112 : tissu coton sali de cacao
- n° 117 : tissu polyester/coton 65/35 sali de sang, lait et encre de chine.

On a préparé une base détergente ayant la composition suivant (parties en poids)

14 parties de surfactant anionique (n-alkylbenzenesulphonate)

4 parties de surfactant non-ionique (alcool gras polyethoxylé)

2 parties de savon

30 parties de tripolyphosphate sodique

10 parties de silicate sodique

30 parties de sulfate sodique

10 parties de carbonate sodique

0,8 parties d'agent de blanchiment optique

On a dilué cette base détergente à raison de 4 g par litre d'eau distillée. Le pH de ce mélange était de 9,4.

A partir de ce mélange, on a préparé deux solutions, l'une (exemple 5a) contenant 2,5 g/l de la poudre obtenue à l'exemple 1, l'autre (exemple 5b) contenant 0,6 g/l de la poudre obtenue à l'exemple 3. On a porté ces solutions à 30°C, et on a mesuré l'activité lipasique résiduelle en fonction du temps de contact, par la méthode colorimétrique :

|  | au départ | après 10 min. | 20 min. | 30 min. |
|---|---|---|---|---|
| exemple 5a | 100% | 100% | 95% | 91% |
| exemple 5b | 100% | 100% | 95% | 91% |

Exemple 6

L'activité enzymatique de la lipase de l'invention en solution détergente a été évaluée sur des tissus standards EMPA (Eidgenössische Materialprüfungs- und Versuchsanstalt, St. Gallen, CH) avec salissures normalisées :
- n° 104 : tissu polyester/coton 65/35 sali d'huile d'olive et noir de carbone
- n° 112 : tissu coton sali de cacao
- n° 117 : tissu polyester/coton 65/35 sali de sang, lait et encre de chine.

Les essais de lavage ont été réalisés dans des fioles cylindroconiques placées dans un incubateur orbital à 37°C.

Trois coupons du même tissu (9 x 9 cm) préalablement numérotés et de réflectance initiale connue, ont été introduits dans une fiole de 500 ml contenant 100 ml de la solution détergente à tester, préparée au dernier moment et dont 5 ml ont été prélevés pour dosage enzymatiques au temps initial.

Après agitation orbitale à 200 RPM durant 30 minutes, les tissus ont été rincés par 3 x 250 ml d'eau distillée et séchés par repassage tandis que les activités enzymatiques résiduelles ont été dosées dans la solution après lavage.

La réflectance moyenne est calculée et comparée d'une part à celle du tissu non lavé et d'autre part à celle d'un échantillon de référence lavé en parallèle dans une solution sans enzyme. On a observé les augmentations de réflectances suivantes :

| Tissu n° | 104 | 112 | 117 |
|---|---|---|---|
| lipase de l'Ex. 1 | + 5,6 | + 10,9 | + 8,4 |
| lipase de l'Ex. 3 | + 9,3 | + 12,1 | + 6,0 |

Exemple 7

On a évalué l'activité enzymatique de la lipase de l'invention en solution détergente contenant un oxydant, en suivant la procédure décrite à l'exemple 6 tout en ajoutant 1 g/l de perborate de sodium aux solutions de lavage. On a observé les augmentations de réflectance suivantes :

| Tissu n° | 104 | 112 | 117 |
|---|---|---|---|
| Lipase de l'Ex. 1 | + 5,4 | + 27,7 | + 5,7 |
| lipase de l'Ex. 3 | + 9,7 | + 6,7 | + 2,9 |

Exemple comparatif A

On a utilisé une lipase, vendue sous le nom AMANO P, produite par la souche <u>Pseudomonas fluorescens</u> IAM 1057, plus amplement décrite dans la demande de brevet JP-53/20487-A de AMANO PHARMACEUTICAL CO LTD; Nagoya, Japon. En solution à 1 mg/ml, cette lipase a une activité correspondant à 2300 UE colorimétriques ou 25 UE titrimétriques.

On a observé la stabilité de cette lipase à 0,5 g/l en solution détergente en suivant la procédure de l'exemple 5 :
- au départ : 100%
- après 10 min. : 84%
- après 20 min. : 63%
- après 30 min. : 46%

L'efficacité de cette lipase a été évaluée en solution détergente en suivant la procédure de l'exemple 6. On a observé les variations de réflectance suivantes :

| Tissu n° | 104 | 112 | 117 |
|---|---|---|---|
| AMANO P | - 1,8 | + 2,5 | + 3,0 |

En présence d'oxydant (procédure de l'exemple 7), on a observé les variations de réflectance suivantes :

| Tissu n° | 104 | 112 | 117 |
|---|---|---|---|
| AMANO P | + 2,4 | -4,3 | + 0,1 |

Exemple 8 et exemple comparatif B

On a utilisé la lipase de l'exemple 2 et, à titre de comparaison, une lipase commercialisée pour usage en détergence par NOVO sous le nom de Lipolase™ 30T.

On a préparé une solution à 0,5 g de lipase par litre dans une solution détergente standard "ECE 77", conçue pour un test ECE de résistance de couleurs et ayant la composition suivante :

| | |
|---|---|
| - n-alkylbenzene sulfonate en $C_{11.5}$ | 0,29 g |
| - alcool de suif ($C_{14}$) éthoxylé | 0,105 g |
| - sel sodique d'acide gras (80% $C_{18}$ - $C_{22}$) | 0,125 g |
| - triphosphate sodique | 1,59 g |
| - silicate sodique ($SiO_2/Na_2O = 3,3$) | 0,27 g |
| - silicate de magnésium | 0,07 g |
| - carboxyméthylcellulose | 0,04 g |
| - ethylènediamine tétraacétate sodique | 0,01 g |
| - sulfate sodique | 0,765 g |
| - perborate sodique | 1,00 g |
| - eau | q.s.p. 1 litre |

On a mesuré l'activité colorimétrique de chaque lipase à 30°C dans cette solution, rapportée à celle de la même lipase mesurée en solution aqueuse dans les mêmes conditions :

| Activité colorimétrique relative | Lipase de l'ex. 2 | Lipolase 30T |
|---|---|---|
| a) en solution aqueuse | 100% | 100% |
| b) en solution détergente | | |
| t - 0 min | 100% | 10% |
| t - 10 min | 99% | 0% |
| t - 20 min | 95% | 0% |
| t - 30 min | 88% | 0% |

On a répété ces expériences en omettant le perborate sodique de la composition, sans observer de modification significative de la stabilité.

**Revendications**

1. Lipase caractérisée en ce qu'elle peut être produite par la souche <u>Pseudomonas</u> déposée le 8 février 1988 sous le numéro CBS 134.88 au Centraalbureau voor Schimmelcultures à Baarn, Pays-Bas.

2. Protéase caractérisée en ce qu'elle peut être produite par la souche <u>Pseudomonas</u> déposée le 8 février 1988 sous le numéro CBS 134.88 au Centraalbureau voor Schimmelcultures à Baarn, Pays-Bas.

3. Additif enzymatique pour détergent, caractérisé en ce que son principal composant actif est la lipase selon la revendication1.

4. Additif enzymatique pour détergent selon la revendication 3, caractérisé en ce qu'il contient en plus une protéase.

5. Additif enzymatique pour détergent selon la revendication 4, caractérisé en ce que la protéase est celle de la revendication 2.

6. Utilisation de la souche <u>Pseudomonas</u> déposée le 8 février 1988 sous le numéro CBS 134.88 au Centraalbureau voor Schimmelcultures à Baarn, Pays-Bas, pour produire une lipase et/ou une protéase.

7. Compositions détergentes contenant la lipase selon la revendication 1.

8. Compositions détergentes contenant la protéase selon la revendication 2.

9. Compositions détergentes contenant un additif enzymatique selon l'une quelconque des revendications 3 à 5.

10. Procédé de lavage utilisant les compositions détergentes selon l'une quelconque des revendications 7 à 9.

**Patentansprüche**

1. Lipase, dadurch gekennzeichnet, daß sie durch den Stamm <u>Pseudomonas</u> erzeugt werden kann, der am 8. Februar 1988 unter der Nummer CBS 134.88 beim Centraalbureau voor Schimmelcultures, Baarn, Niederlande hinterlegt worden ist.

2. Protease, dadurch gekennzeichnet, daß sie durch den Stamm <u>Pseudomonas</u> erzeugt werden kann, der am 8. Februar 1988 unter der Nummer CBS 134.88 beim Centraalbureau voor Schimmelcultures, Baarn, Niederlande hinterlegt worden ist.

3. Enzymatischer Zusatz für Reinigungsmittel, dadurch gekennzeichnet, daß sein aktiver Hauptbestandteil die Lipase nach Anspruch 1 ist.

4. Enzymatischer Zusatz für Reinigungsmittel nach Anspruch 3, dadurch gekennzeichnet, daß er weiter eine Protease enthält.

5. Enzymatischer Zusatz für Reinigungsmittel nach Anspruch 4, dadurch gekennzeichnet, daß die Protease die nach Anspruch 2 ist.

6. Verwendung des Stammes Pseudomonas, der am 8. Februar 1988 unter der Nummer CBS 134.88 beim Centraalbureau voor Schimmelcultures, Baarn, Niederlande hinterlegt worden ist, um eine Lipase und-/oder eine Protease zu erzeugen.

7. Reinigungszusammensetzungen, die die Lipase nach Anspruch 1 enthalten.

8. Reinigungszusammensetzungen, die die Protease nach Anspruch 2 enthalten.

9. Reinigungszusammensetzungen, die einen enzymatischen Zusatz nach irgendeinem der Ansprüche 3 bis 5 enthalten.

10. Verfahren zum Waschen unter Verwendung der Reinigungszusammensetzungen nach irgendeinem der Ansprüche 7 bis 9.


## Claims

1. Lipase characterised in that it can be produced by the Pseudomonas strain deposited at the Centralbureau voor Schimmelcultures in Baarn, The Netherlands on 8 February 1988 under the number CBS 134.88.

2. Protease characterised in that it can be produced by the Pseudomonas strain deposited at the Centralbureau voor Schimmelcultures in Baarn, The Netherlands on 8 February 1988 under the number CBS 134.88.

3. Enzymic additive for detergent characterised in that its principal active constituent is the lipase according to claim 1.

4. Enzymic additive for detergent according to claim 3, characterised in that it moreover contains a protease.

5. Enzymic additive for detergent according to claim 4, characterised in that the protease is that of claim 2.

6. Use of the Pseudomonas strain deposited at the Centralbureau voor Schimmelcultures in Baarn, The Netherlands on 8 February 1988 under the number CBS 134.88, to produce a lipase and/or a protease.

7. Detergent compositions containing the lipase according to claim 1.

8. Detergent compositions containing the protease according to claim 2.

9. Detergent compositions containing an enzymic additive according to any one of claims 3 to 5.

10. Washing process using the detergent compositions according to any one of claims 7 to 9.